Europäisches Patentamt

European Patent Office

Office européen des brevets

(11) Publication number: **0 517 529 A2**

# EUROPEAN PATENT APPLICATION

(21) Application number: **92305155.1**

(51) Int. Cl.⁵: **A61C 8/00, A61B 17/14**

(22) Date of filing: **05.06.92**

(30) Priority: **07.06.91 JP 136566/91**
**14.02.92 JP 28064/92**

(43) Date of publication of application:
**09.12.92 Bulletin 92/50**

(84) Designated Contracting States:
**CH DE FR GB IT LI**

(71) Applicant: **Inoue, Kiyoshi**
**3-16-7 Kami-Yoga, Setagayaku**
**Tokyo(JP)**

(72) Inventor: **Inoue, Kiyoshi**
**3-16-7 Kami-Yoga, Setagayaku**
**Tokyo(JP)**

(74) Representative: **Enskat, Michael Antony Frank**
**Saunders & Dolleymore 9, Rickmansworth**
**Road**
**Watford Hertfordshire WD1 7HE(GB)**

(54) Jawbone cutting tool and apparatus.

(57) A dental bone cutting tool (15) for machining recesses for anchoring a dental implant is disclosed. The implant comprises a pair of blades juxtaposed and coupled in parallel with each other and a head for supporting a denture. The head is supported by the two blades and has a base surface continuous with the blades. The tool (15) comprises a pair of large diameter cutter disks (16) constituting each a circular saw blade with a peripheral cutter edge and securely mounted on a hollow rotary shaft (18) for cutting the two parallel grooves for accepting the two parallel blades, respectively. Smaller diameter cutter disks (17) are also clamped in parallel together and mounted on the rotary shaft, and so numbered and sized as to collectively constitute a unitary rotary cutter for cutting a recess so sized and configured as to accept at least a portion of the base surface of the implant head in the jawbone. A coolant is supplied through the hollow rotary shaft and via the cutter disks into the cutting zone.

FIG. 9

The present invention relates to dental implantology and, more particularly, to a jawbone cutting tool and apparatus for forming recesses in a jawbone, i. e. maxilla or mandibular, for accepting a dental implant therein, as well as a method of the implantation of a dental implant in the jawbone. The invention also relates to an improved denial implant. More generally, the invention relates to a bone cutting tool, apparatus and method for a surgical operation.

A dental implant commonly comprises an root portion to be embedded in the jawbone and a head or post portion supported by the root portion and adapted to carry a denture, or a dental crown or bridge. It is important that the root portion, advantageously formed by a single or a plurality of thin, flat plate or blade-like members, establish, when inserted into and embedded in, the jawbone, a strong connection with the bone by intimately adhering thereto or tenaciously combining therewith, thus capable of stably and firmly holding a denture on or over the head portion supported by the root portion. It being that teeth or denture must withstand an occlusal force as high as 35 to 70 kgf, a dental implant, when poorly anchored, tends to loosen or displace and hence cannot sustain a long time period of use.

In German Utility Model G 90 05 790.2, there is disclosed an improved dental implant with certain aesthetic features. The implant there disclosed has improved utility features as well. It comprises a pair of parallel profiled plate or blade members constituting its root portion for the implantation in the jawbone and one or more posts connected or integral with the blades for securely holding a denture or denture supporting structure, thereby firmly anchoring the denture in the bone of a maxilla or mandible. Here, the integration or connection of the or each post with the blades is such that its base portion is formed to project between the blades in the form of an arch in their upper region, when the blades are embedded in the jawbone, to lie below the surface of the mandibular or maxillary bone crestal ridge. The height of this downward projection is preferably not greater than one half of the height (maximum) or vertical width (maximum) of the blades as shown. It has been found that such a blade-type implant, when properly implanted, provides an improved and extremely tenacious and stable connection with the jawbone, this being so even where there is a severe limitation in the implantable depth and hence height of blades in the jawbone.

In order for this blade-type implant to be properly anchored, peculiar recesses must be cut in, the jawbone so that the blades may be properly and fittedly accommodated and stay with stability therein. A tool for cutting these recesses make use of a pair of parallel and large-diameter edged cutter disks and a small-diameter edged cutter member, these cutter disks and member being securely and co-axially or a common axle driven by means of a motor. The large-diameter cutter disks serve to cut a pair of parallel grooves for accepting the parallel implant blades. The small-diameter cutter member is made and arranged to cut a groove for accepting the base portion of the post or each post supported by the parallel blades which portion is arranged to be integral therewith so as to project into therebetween. A cutting tool of the type described above is shown and described generally in Japanese Patent Publication No. H03-131249 dated June 4, 1991.

While the cutting tool described and a dental apparatus which makes use thereof are found to be generally useful, there exists what is still desirable of its cutting performance and quality. Furthermore, there has been found a significant lack of versatility of its applications.

Accordingly, it is a principal object of the present invention to provide an improved jawbone cutting tool and apparatus which can be used to form recesses in the jawbone for accepting parallel blades or plate of a dental implant as described and an arc-rimmed base portion of the blade-connected or -integral post of a dental implant as described and which can be applied with considerable versatility for implantation.

Another object of the present invention is to provide an improved jawbone cutting tool and apparatus whereby recesses or grooves of individual functions can be formed quite accurately, reliably machined for accepting a dental implant as described and of varied types effectively and efficiently, and with the net result that the implant may anchor in the jawbone with extreme secureness and without a loss of stability.

These and other objects are attained in accordance with to present invention by providing, in a first aspect thereof, a jawbone cutting tool for machining recesses for anchoring a dental implant in a jawbone in which the implant comprises a plurality of blade members juxtaposed and connected in a parallel relationship with one another and a head member adapted to support a denture or a denture supporting structure and supported by the blade members and having a base portion continuous therewith, the tool comprising: a plurality of relatively large diameter cutter disks constituting each a circular saw blade with a peripheral cutter edge and mounted securely on a rotary shaft for cutting a plurality of recesses for accepting the said implant blade members in the jawbone; and a predetermined number of relatively small diameter cutter disks clamped in parallel together and mounted on the said rotary shaft and which are so numbered and individually sized as to collectively constitute a substantially unitary saw blade to provide a profiled cutting surface with their peripheral cutting edges for cutting a recess so sized and configured as to accept at least a portion of the base surface of the implant head member in the jawbone.

The invention also provides, in a second aspect thereof, a dental cutting tool for machining recesses for anchoring a dental implant in a jawbone in which the implant comprises a blade member and at least one head member adapted to support a denture or denture supporting structure and supported by the blade member, and having a base surface continuous therewith, the tool comprising a relatively large diameter cutter disk constituting a circular saw blade with a peripheral cutting edge and mounted securely on a rotary shaft for cutting a recess for accepting the said implant blade member in the jawbone; and a predetermined number of relatively small diameter cutter disks clamped together and with the relatively large diameter cutter disk and mounted an the rotary shaft as being divided on both sides of the relatively large diameter cutter disk, the relatively small diameter cutter disks being so numbered and individually dimensioned as to collectively constitute a substantially unitary saw blade with their peripheral cutting edges for cutting a recess so dimensioned and configured as to accept at least a portion of the base surface of the implant head member in the jawbone.

The relatively small diameter cutter disks may be thin, and unit cutter disks of an equal thickness may be prepared in a set with various diameters.

The rotary shaft may be hollow to provide a fluid passage for flushing a coolant in a cutting zone constituted by the peripheral cutting edges.

The implant blade member(s), and also unit cutter disks, may be composed of a bio-compatible substance and the large diameter disks may individually be a thin, annular layer of a hard material containing the bio-compatible substance coated thereon along a region of the peripheral edge. The bio-compatible substance consists substantially of pure titanium or a titanium alloy and the material preferable consists substantially of titanium nitride or titanium carbide and the cutter disks may individually a base body or substrate consisting of a soft stainless steel.

The invention provides, in a third aspect thereof, a dental bone cutting apparatus for machining recesses in a jawbone for anchoring a dental implant comprising at least one blade member, and at least one head member supporting a denture or a denture supporting structure, the at least one head member having a predetermined base surface profile and being supported by the at least one blade member and continuous therewith, the apparatus comprising: at least one relatively large diameter cutter disk constituting a first circular saw blade means with peripheral cutter edges thereof for cutting the at least one implant blade member; a plurality of relatively small diameter cutter disks clamped together and with the at least one implant blade member; a plurality of relatively small diameter cutter disks clamped together and with the at least one relatively large diameter cutter disk on a rotary shaft, and so numbered and dimensioned as to constitute a substantially unitary rotary saw blade with their peripheral cutting edges for cutting thereby a recess so dimensioned and configured as to accept the said predetermined profile base surface of the at least one head member; the rotary shaft being hollow and having an internal fluid passage; drive means for rotating the hollow rotary shaft; and a coolant supply for supplying a coolant under pressure into the fluid passage of the rotary shaft, when rotated at a high speed, for flushing the coolant into a bone cutting zone constituted by the cutter saw blades while in rapid rotation.

The invention also provides, in a forth aspect thereof, a bone cutting apparatus for at least one recess of a predetermined profile in a patient bone in a surgical operation, comprising: a plurality of thin, unit cutter blades clamped in parallel together and mounted on a hollow rotary shaft, the unit cutter blades being so numbered and individually dimensioned as to collectively constitute a substantially unitary rotary saw blade with a cutting profile substantially complementary to the predetermined profile; the hollow rotary shaft internally having a fluid passage communicating with a cutting zone constituted by the cutter blades; drive means for rotating the hollow rotary shaft and thereby the substantially unitary rotary blade for cutting the recess; and means for supplying a fluid coolant through the fluid passage into the cutting zone.

The invention also provides, in a fifth aspect thereof, a method of cutting a contour of a predetermined profile in a workpiece, comprising the steps of: preparing a plurality of thin, unit cutter disks of various diameters; selecting a predetermined set of the unit cutter disks, arranging and clamping them together and upon a rotary shaft in such a manner that the selected, arranged and clamped disks collectively constitute a substantially unitary rotary cutter with a peripheral profile substantially complementary to the said predetermined profile; and rotating the rotary shaft and thereby the clamped disks for cutting the said contour in the workpiece.

The rotary shaft may be hollow and provided with an internal fluid passage communicating with a cutting zone constituted by the disks and the workpiece. The method may further comprise passing a coolant through the fluid passage to flush the cutting zone therewith.

The invention also provides, in a further aspect thereof, a bone cutting tool comprising a rotary cutter disk composed of a soft material and having an added annular layer of a hard material containing a bio-compatible substance coated along a peripheral cutting regions of the disk. Such a substance may be titanium or a titanium alloy. The hard material may consist of titanium nitride or titanium carbide, or a continuous body of titanium nitride or titanium carbide and fine particles of diamond or CBN distributed therein.

The disk is preferably formed with a plurality of fluid passages formed therein or thereon for guiding a coolant into a bone cutting zone.

The invention, in still a further aspect thereof, provides a dental implant which comprises; a blade-type root portion comprising a pair of blade members juxtaposed in parallel with each other and connected by a head portion, the blade members having a vertical height $a$; the head portion having a lower end with a base contour of a predetermined profile, the head lower end extending into between the blade members with a length of extension $b$, wherein $b$ is not grater than one half of $a$.

At this point, it should be noted that the terms "upper", "lower", "top", "bottom" or the like are used throughout the specification with reference to the direction of depth, and are not necessarily intended to be used with reference to the direction of gravity.

These and other objects, features and advantages of the present invention will become more readily apparent from a reading of the following description taken with reference to the accompanying drawings in which:

FIG.1 is a frontal elevational view of a parallel-blade type dental implant with which the present invention is concerned;

FIG.2 is a top plan view thereof;

FIG.3 is a bottom plan view thereof;

FIG.4 is a side elevational view thereof, thereof, the opposite side being a mirror image;

FIG.5 is a cross-sectional view thereof, taken on line V-V in FIG.1;

FIG.6 is a cross-sectional view thereof, taken on line VI-VI in FIG.1;

FIG.7 is a cross-sectional view thereof, taken on line VII-VII in FIG.1;

FIG.8 is a cross-sectional view thereof, taken on line VIII-VIII in FIG.1;

FIG.9 is a top perspective view thereof;

FIG.10 is a perspective view of a jawbone diagrammatically illustrating recesses formed therein to accept the implant illustrated in FIGS.1-9;

FIG.11(a) is a frontal elevational view diagrammatically illustrating another improved blade-type implant according to the present invention;

FIG.11(b) is a side elevational view of the implant of FIG.11a, the opposite side being a mirror image;

FIG.11(c) is a bottom plan view of the implant of FIGS.11a and 11b;

FIGS.12(a) and 12b are frontal elevational view, respectively, diagrammatically illustrating one embodiment of a cutting tool according to the invention;

FIG 13 is a top plan view diagrammatically illustrating another form of the cutting tool embodying the present invention;

FIG.14 is a frontal view, partly elevational and partly in section, diagrammatically illustrating the cutting tool shown in FIG.12 or 13 while in the process of cutting recesses in the jawbone according to the present invention;

FIG.15 is a top plan view diagrammatically illustrating such recesses cut in the jawbone;

FIG.16 is a side view, partly elevational an partly in section, diagrammatically illustrating a cutting apparatus that embodies the principles of the present invention;

FIG.16(a) is an enlarged view, partly elevational and partly in section, of the rotary drive shaft shown in FIG.16;

FIGS.16(b) and 16(c) are side elevational views diagrammatically illustrating a cutter disk provided with coolant flushing passages;

FIG.17 is a top plan view diagrammatically illustrating another farm of the cutting tool according to the invention;

FIGS.18(a), 18(b) and 18(c) are a front elevational, a side elevational and a top plan view, respectively, diagrammatically illustrating a single, double-arc profiled blade-type implant which can be implanted using a cutting took and apparatus according to the present invention;

FIG.19 is a top plan view diagrammatically illustrating a cutting tool adapted to cut recesses for the implant shown in FIG.18(a)-(c).

FIGS.20(a) and 20(b) are frontal elevational views diagrammatically illustrating modified cutter disks in the practice of the invention;

FIGS.21(c), 21(b), 21(c) and 21(d) are top plan views diagrammatically illustrating further modified cutter assemblies for the practice of the present invention;

FIGS.22(a), 22(b), 22(c) and 22(d) are diagrammatic elevational views illustrating the prior art;

FIGS.23(a), 23(b) and 23(c) are front elevational, top plan and side elevational views, respectively, diagrammatically illustrating a further form of the profiled parallel-blade type dental implant to which the principles of the present invention may apply; and

FIGS, 24(a) and 24(b) are front elevational and top plan views, respectively, diagrammatically illustrating a still further form of dental implants of the class described which can be subject to the practice of the present invention.

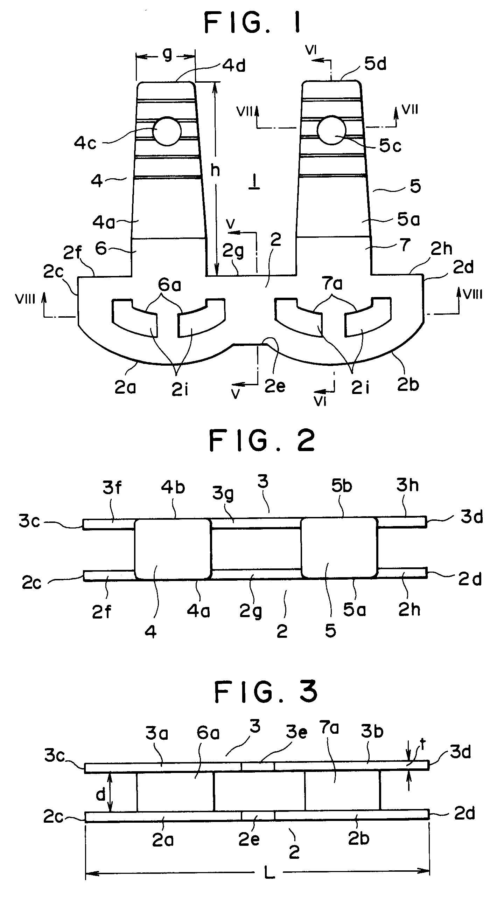

In FIGS.1-9 there is shown a dental implant as disclosed in German Utility Model G 90 05 790.2 and designated here generally by a numeral 1. The implant 1 comprises a pair of parallel, vented plates or blades 2 and 3 with an identical rim or peripheral shape and consists of a bio-compatible material such as pure titanium or a titanium alloy. Thus, each blade 2, 3 has a pair of co-planar arc-formed rims 2a and 2b; 3a and 3b, and is flanked with a pair of vertically straight outer walls 2c and 2d; 3c and 3d, the arc-formed rims being interconnected by horizontally straight contours 2e and 3e, respectively. The upper contours of the two blades 2 and 3 horizontally extend and are co-planar, and each constituted by three straight portion 2f, 2g and 2h; 3f, 3g and 3h. The blade portions 2, 3 further include a series of vents 2i; 3i, each extending, here, generally in alignment with the adjacent rims 2a and 3b of the blades 2, 3. The vents 2i, 3i are provided to allow bone growth therethrough in a healing stage after implantation.

The head portion of the implant 1 comprises a pair of truncated-pyramidal posts 4 and 5 adapted to carry a denture or denture supporting structure (not shown). The posts 4 and 5 are shown as extending in an axially parallel relationship from the blade-formed portions 2 and 3 of the implant 1. Here, the heads or posts 4 and 5 are each tapered upwardly to facilitate the mounting of a denture or denture supporting structure. Their front and rear side surfaces 4a, 5a; 4b, 5b downwards lie generally co-planar with the front and rear side surfaces of the parallel blades 2 and 3, respectively. Each post 4, 5 extends via a base portion 6, 7 forming a lower part thereof and connected or integral therewith from the blades 2 and 3 which are in turn connected or integral with the base portions 6 and 7. The base portions 4c and 5c each have its lower surface which is here again in the form of an arc, extending into between the blades 2 and 3. It is thus seen that the base portions 6 and 7 of the posts 4 and 5 in the regions of their lower surfaces 6a and 7a are sandwiched between the blades 2 and 3 to be inserted into the jawbone, viz. a region of the alveolar bone of the maxilla or mandible of a patient. This means that when the implant 1 is properly implanted, the base portions 6 and 7 of the posts 4 and 5 are embedded in the alveolar bone with the lower surfaces 6a and 7b lying below the surface of alveolar bone.

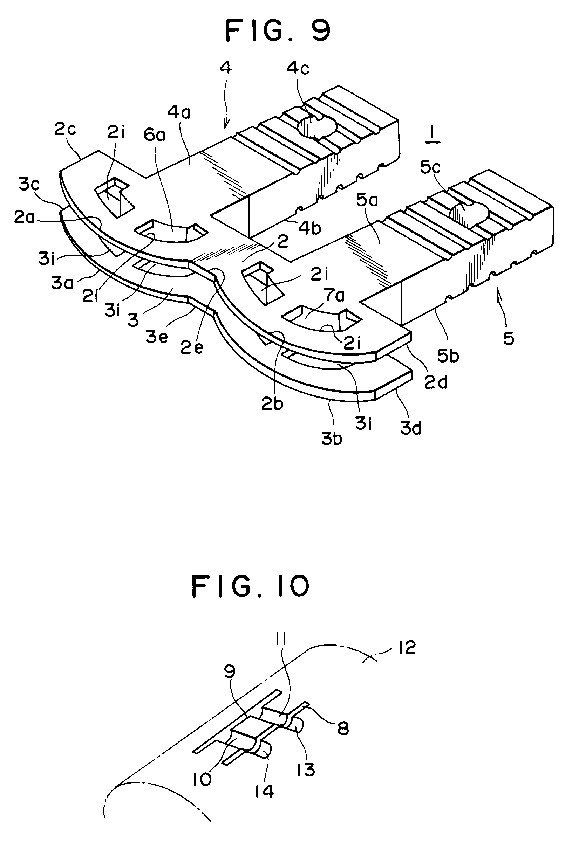

The function of the base portion 6, 7 of each denture carrying post 4, 5 with its shaped (arc-formed) contour 6a, 7a is to properly and accurately position the implant 1 with respect to the jawbone when grooves are cut therein for accepting the blades 2 and 3. FIG.10 shows grooves or recesses 8, 9, 10 and 11 cut in the jawbone 12 and designed to fittedly accept the root portions and head base portions of the implant 1, thus grooves 8 and 9 for blades 2 and 3, respectively, and grooves 10 and 11 for the base portions 6 and 7 of the posts 4 and 5, respectively.

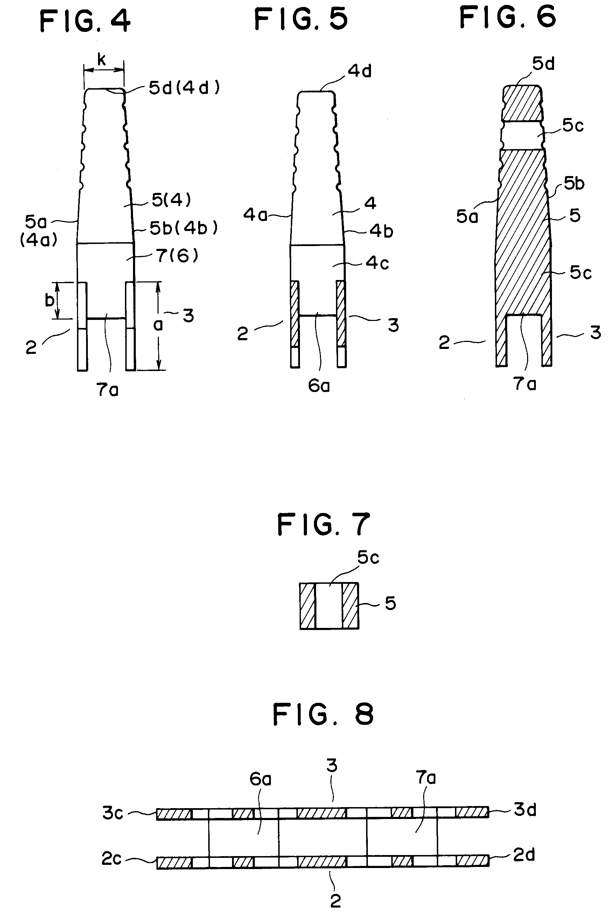

It has been found to be important that the blade portions 2, 3 and the head base portions 6, 7 have their heights a and b, respectively, as shown in FIG.4 such that the height b (of the head base portions 6, 7) is not in excess of one half (1/2) of the height a (of blades 2, 3). When these elements of the implant 1 are so designed, it has been found that a highly accurate positioning of the implant 1 and hence a denture is established with respect the jawbone 7 prior to anchorage therein and a highly stable and durable implantation can be achieved and that this is even so where the implantable blade height is relative low.

The head portions 4 and 5 as shown further include openings 4c and 5c, respectively, in order to facilitate insertion of the implant 1 in the jawbone 12 and, when needed, retraction of the implant therefrom.

In a typical example of the implant 1 shown in FIG.1-9 and described, each blade 2, 3 has its length (L) of 18 to 20 mm and its thickness (t) of 0.4 to 0.5 mm. The blades 2 and 3 are spaced apart with a distance (d) of 1.5 to 2.5 mm. The heads 4 and 5 have each a height (distance between its top 4d, 5d and the blade upper surface 2g) (h) of 9 to 11 mm, and its top longitudinal width (9) of 2.5 to 3.5 mm and top transverse width (k) of 1.5 to 2.5 mm. The heads 4 and 5 are interspaced at their bases with a distance (m) 9 to 10 mm. The blades 2 and 3 each have a height (a) of 4 to 6 mm whereas the head base portions 4 and 5 which project or extend into between the blades 2 and 3 each has a height of projection or length of extension (b) of 2 to 2.5 mm with the aforementioned relationship: b is not greater than 1/2a or a/b is not less than 2.

The implant 1 or any other form of the implants hereinafer described is preferably cut and formed by a wire-cut or traveling-wire EDM process.

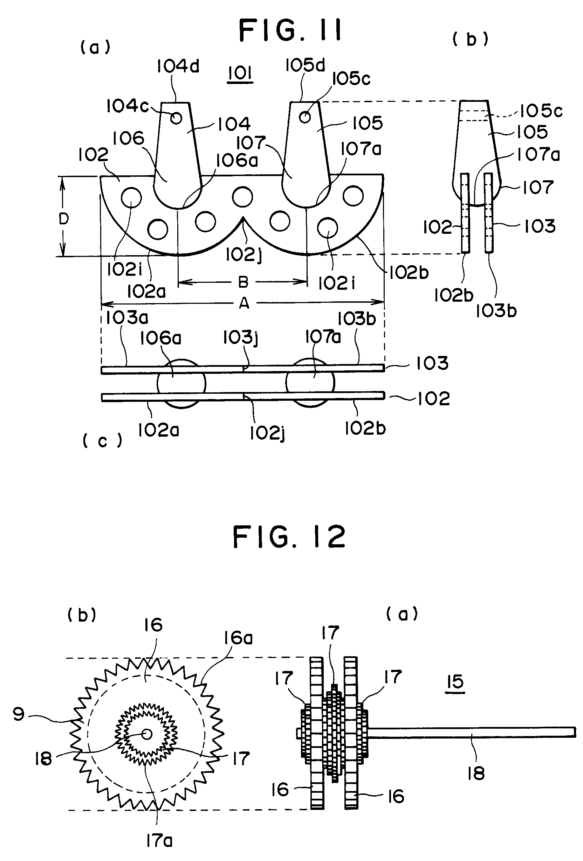

FIGS.11(a), 11(b) and 11(c) show another form of the dental implant 101 practicing the present invention. The implant 101, consisting of a bio-compatible substance such as pure titanium or a titanium alloy, has a root portion which, as seen from FIG.11(b), comprises, here again, a pair of parallel vented plates or blades 102 and 103 with their rims or peripheries of an identical profile with each rim or periphery comprising a pair of arcs 102a and 102b, 103a and 103b connected together at their junction 102j, 103j to form a co-planar double-arc profiled contour. The vents formed in each blade 102, 103 as shown at 102i in FIG.11(a) are circular but may be shaped otherwise, e.g. as shown in FIG.1, and have a function already described in connection therewith.

The head portion of the implant 101 includes a pair of truncated conical posts 104 and 105 for carrying a denture or denture supporting structure (not shown). The posts 104 and 105 extend in an axially parallel relationship from the blade-formed root portions 102 and 103 of the implant 101. The posts 104 and 105 are each tapered towards their tops 104d and 105d and are each bulged at or towards their base portions 106, 107 shown as integral therewith, so that they have their lower surfaces 106a and 107a each in a semi-spherical shape. Thus, these semi-spherical base surfaces 106a and 107a are each an arc in a vertical cross-section which is concentric or substantially concentric with the elemental arc 102a, 102b, 103a, 103b of the double-arc profiled blade 102, 103. Specifically, as shown in FIG.11(c), each head base portion 106, 107 is circular in a horizontal cross-section and, with its diameter greater than the inter-spacing of the blades 102 and 103, is bulged outsides these parallel implant members. The so-formed head base portions 106 and 107 function to properly and accurately position the implant with respect to the jawbone when grooves are cut therein for accepting the blades 102 and 103.

FIGS.12(a) and 12(b) show a cutting tool 15 adapted to simultaneously effect a cut of grooves for accepting the blades 102 and 103 and a groove cut for accepting each head lower base portion 106, 107. The tool 15 makes use of a plurality of unit cutter disks which, in this embodiment, comprises a first class of unit disks 16 of a same thickness and a same relatively large diameter for cutting grooves for the blades 102 and 103 and a second class of unit disks 17 of a same thickness and various relatively small diameters which are stacked and clamped together with the first class of unit disks 16 and securely mounted on a rotary shaft 18 to constitute a substantially, unitary rotary cutter with a peripheral cutting profile adapted to cut in the jawbone a groove or recess for accepting the semi-spherical surface 106a, 107a of each head base 106, 107. Each cutter disk 16, 17 has on its periphery a cutting edge 16a, 17a.

For example, the relatively large-diameter cutter disks 16 have a thickness of 0.45 to 0.5mm corresponding to the width of each blade 102, 103 and a radius which may correspond to the radius of each elemental arc 102a, 102b; 103a, 103b. The disks 16 when securely mounted on the rotary shaft 18 has an inter-spacing corresponding to the inter-spacing of the parallel blades 102 and 103. The small-diameter unit cutter disks 17 may each have a thickness of 0.2mm. Ones with varying diameters are arranged and securely mounted on the rotary shaft 18 to provide a collective cutting contour which is, in the embodiment shown, adapted to cut a generally semi-spherical recess corresponding to the semi-spherical surface 106a, 107a of the head supporting base portion 106, 107.

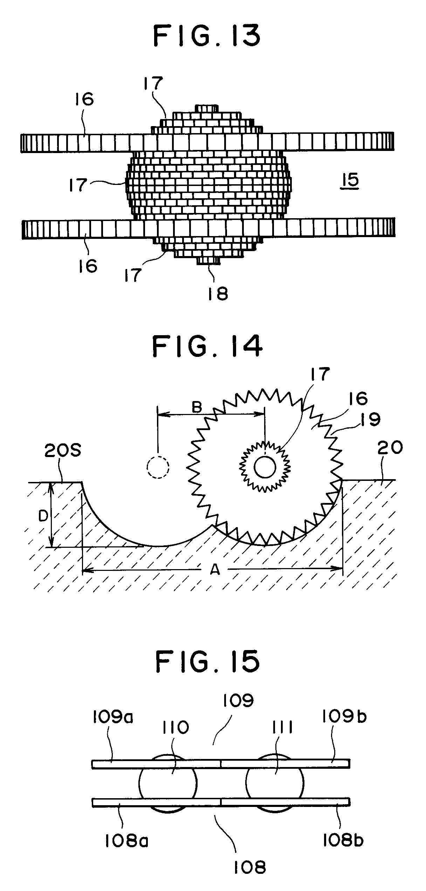

In the assembled cutter shown in FIG.13, the number of unit cutter disks 17 of the second class between and each outside of the two relatively large diameter and a relatively thick cutter disks of the first class are increased. Thus, in the embodiment of FIG.13, the large-diameter and thick cutter disks 16 have a thickness of 0.5mm each and are mounted with an inter-spacing of 2mm securely on the rotary shaft 18. Between these two disks there are ten relatively small diameter and thin cutter disks of a thickness of 0.2mm and varying diameters, viz. 3.8mm, 3.7mm, 3.6mm, 3.4mm and 3.2mm from the center outwards, stacked securely mounted on the rotary shaft 18. Each outsides of the large-diameter disks 16 there are four thinner unit cutter disks of a thickness of 0.1mm and varying diameters, viz. 2mm, 1.5mm. 1mm and 0.6mm, stacked and securely mounted on the rotary shaft 18. These thin and thinner unit disks so assembled provide a rotary cutting surface corresponding to that of a spherical rotary cutter of a diameter of 3.8mm.

It is desirable and most often important that each of cutter disks 16 and 17 along its peripheral cutting edge has an added hard layer 19 of TiC or TiN (sometimes tungsten carbide) so that each disk itself may consist of a soft stainless steel. Titanium and stainless steel are advantageous in the dental environments with which the present invention is concerned. The added layer 19 is provided on the disk by CVD (chemical vapor deposition), preferably plasma CVD or PVD (physical vapor deposition) techniques. In the plasma process, the cathode is constituted by titanium for vaporization which is combined with carbon or nitrogen from a plasma-or thermally decomposable gas furnished into the reaction zone, to form a coating of TiC or TiN on a workpiece. The TiC or TiN layer 19 may alternatively be provided along the peripheral cutting edge of the disk 16, 17 by the spark deposition process using a TiC or TiN composed electrode. For spark deposition techniques, reference is made, for example, to US Patent No.3,741,426.

FIG.14 shows a cutting tool assembly 15 as afore-described while being in the process of cutting recesses in a jawbone 20. In the process, the bone 20 is exposed upon incision of the overlying gum at a properly chosen implant recipient site. A pair of relatively large diameter disks or saw blades 16, 17 of the cutter tool 15 first cut vertically into the bone 20 and then proceed until a depth of cut D is attained which is necessary and sufficient to accept the blades 102, 103 (2, 3) so that their upper surfaces coma just below the bone level 20S, thus completing the first step of the process designed to cut grooves for the first blade portions 102a and 103a. Towards the end of the first step, the cutter disks 17 assembled act on the bone 20 to cut a recess which is necessary and sufficient to accept therein the first semi-spherical base portion 106 of the head member 104. After the first step is accomplished, the tool 15 is raised vertically and displaced horizontally by a distance B which is equal or corresponds to the distance between the centers of the two arcs forming the profile of each blade 102, 103. Thereafter, the second cutting step follows like the first step to cut now grooves for the second blade portions 102b and 103b and simultaneously a recess for the second semi-spherical base portion 107.

FIG.15 shows overall recesses, consisting of grooves 108a and 109a formed in the first step to accept the first arc blade portions 102a and 103a, grooves 108b and 109b formed in the second step to accept the second arc blade portions 102b and 103b and co-planarly connected with the previous grooves 108a and 109a at their junctions 108i and 109i, respectively and a pair of semi-spherical recesses 110 and 111 formed in the first and second steps, respectively, to accept the semi-spherical base portions 106 and 107, respectively, thus serving to properly and accurately position the denture supporting posts 104 and 105 with respect to the jawbone 20 at the selected implant recipient site. By the formation of auxiliary recesses 110 and 111, it is also possible to prevent the blades 102 and 103 from being embedded to reach an excessive depth which may damage the sub-structure of the jawbone 20, thereby assuring an extremely safe and reliable implantology. With the blade portions 102 and 103 being snugly fitted in and supported by the recesses 108 and 109 (as a close tolerance of 0.05mm on one lateral side) and solidly fixed in a parallel-connected structure and the structure intensified in the jawbone 20, not only is it possible to avoid lateral displacement of the implant but its retentivity after implantation is markedly enhanced.

A dental bridge or artificial crowns may be fixed on the head portions 104, 105 of the implant 101 with a thus enhanced retentivity. The parallel arc profiled root structure of the implant 101 advantageously acts to disperse over the receiving jawbone 20 a pressure arising from an occlusal force acting on the head portion 104, 105 with a value as high as 35 to 70kfg. Thus, there develops a shock absorbing action thereby over the jawbone 20, which serves to remove a feeling of unpleasantness while assuring a stabilized high retentivity since the incipient stage of implantation.

The cutting tool 15 is useful also for cutting recesses adapted to accept an implant with the root portion comprising a pair of single-arc profiled blades parallel connected and a single head supported thereby. For the formation of such recesses, the second step mentioned above is dispensed with. Thus, the first step alone suffices with a single vertical cut to form a pair of parallel grooves for the blades and simultaneously a single recess for the single head base portion.

Depending upon the particular shape of the head portion 104, 105 which is not necessarily truncated-conical as shown in FIG.11 but may be truncated-pyramidal, columnar or any other conventional implant head or post configuration, the shape of the head base portion 106, 107 is not necessarily semi-spherical as shown but may be of planar surface, of any particular curved surface or of any particular surface of projection. When recesses cut in the jawbone 20 need to be shaped so as to complimentarily accept any such particularly shaped surface, the aforementioned feature of the present invention offers the advantage that any overall cutting surfaces for cutting such recesses are provided simply by combining small-diameter unitary cutter disks 17 of varying or identical diameters in a particular set between the large-diameter cutter disks and on one or both outsides thereof upon the rotary shaft 18. It is also possible to provide three distinct cutting surfaces on the rotary shaft 18: a cutting surface the large-diameter cutter disks 16, a cutting surface outside of one of the large-diameter cutter disks 16 and a cutting surface outside of the other large-diameter cutter disks 16.

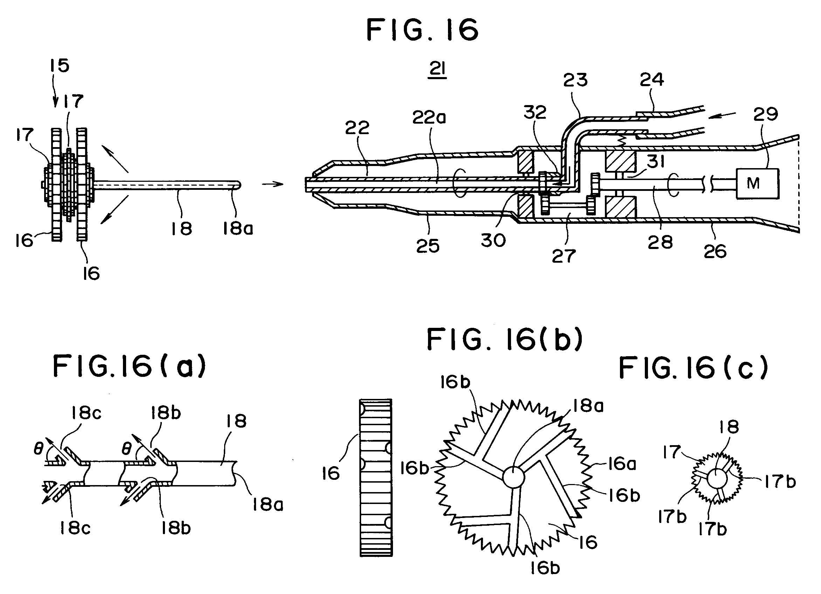

FIG.16 shows a jawbone cutting apparatus 21 according to the present invention, in which the cutting tool 15 previously described is securely chucked. Specifically, the rotary shaft 18 mounting unit disks 16, 17 thereon is chucked or secured into a hollow shaft 22 in engagement therewith. The rotary shaft 18 is here made also to be hollow to provide a fluid passage 18a communicating with a fluid passage 22a internally provided in the hollow shaft 22, which in turn communicates via a duct 23 with a hose 24 that is supplied with a fluid coolant (e.g. a salt solution or a sterilizing gas coolant) from its supply (not shown). The hollow shaft 22 axially extends in a housing or sleeve 25 and slightly projects at its end where the tool shaft 18 is chucked therein. The sleeve 25 has, towards its distal end, a grip-formed housing 26 in which the hollow shaft 22 is mechanically coupled, via a gear transmission 27, with the output shaft 28 of an electric motor 29. The hollow shaft 22 and the motor drive shaft 28 are rotatably journaled on bearings 30 and 31 in the sleeve 25, respectively.

The motor 29 is energized to drive its output shaft 28, causing the hollow shaft 22 to be rotationally driven, via the gear transmission 27, to rotationally drive the rotary shaft 18. Meanwhile the coolant is furnished into the hollow rotary shaft 18 via the fluid inlet 24 and the duct 23. A rotary seal 32 is provided where the fluid duct 23 is mechanically coupled with the hollow rotary shaft 22.

Referring to FIG.16(a), the rotary shaft 18 of the cutting tool 15 is shown as provided with a plurality of fluid outlet openings 18b, 18c, directed towards the peripheral cutting edge portions of cutter disks 16, 17, respectively, for providing the fluid coolant in the cutting zones where these disks perform cutting actions into the jawbone 20. The openings may each have a diameter of, say, 0.3mm and be inclined at an angle of, say, 60 depress away from the axis of the shaft 18.

It has been found to be important that the coolant, preferably, of a salt solution, be supplied into the jawbone cutting areas in order to prevent the thermal damage of the bone being cut which tends to "kill" the live bone or to make it impossible to regenerate during the implant healing stage. By flooding the zones where recesses for the implant blades and recesses for the head base portion are being cut with the fluid coolant to maintain these zones at a low temperature, say, not greater than 50 degrees centigrade when the set of assembled disks 16, 17 is rotated at a speed even as high as 1 to 3 meters/second, that fatal disadvantage is effectively avoided. This effectively allows the cutter rotary speed to be considerably increased, hence a significant reduction of the time of surgery and implantation.

The coolant is flushed into the cutting zones alternatively by using an arrangement as shown in FIGS.16(b) and 16(c) in which coolant passages 16b, 17b are provided in cutter disks 16, 17, which are designed in fluid communication with the internal coolant passage 18a of the hollow rotary shaft 18. The fluid passages 16b, 17b, which may be slits formed on one or both of the side surfaces thereof or inner passages that open at the cutter edge portion 16a, 16b.

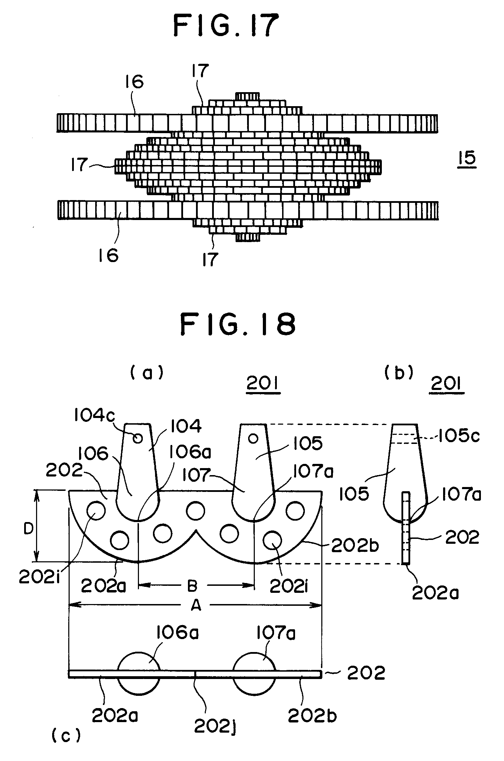

FIG.17 shows an embodiment of the cutting tool 15 in which a set of small, thin diameter unit cutter disks of varying diameters is used to provide an elliptical cutting surface for cutting a elliptical recess for the head base surface which is sometimes required.

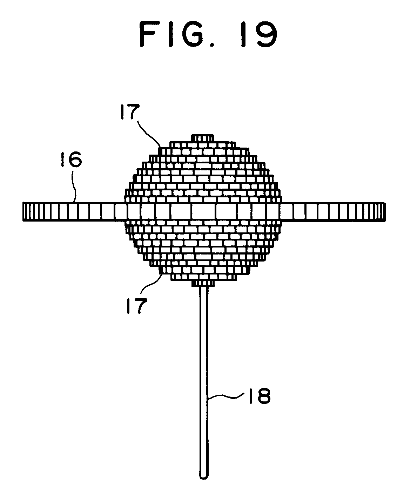

While the foregoing description of features of the present invention is made principally in connection with an implant comprising a pair of parallel blades, it should be understood that the principles of the present invention is applicable to a single blade implant 201 as shown in FIG.18. The implant 201 is shown as comprising, like that shown in FIG 11, a pair of posts or heads 104 and 105 which, however, is here supported by a single blade portion 202. The latter comprises, like each blade portion 102, 103 previously shown and described, a double-arc profiled periphery consisting of a pair of arcs 202a and 202b connected at their junction 202j. The heads 104 and 105 are provided each with the base portion 106, 107 with a semi-spherical surface 106a, 107a as previously shown and described, to accomplish a proper and accurate positioning of the implant 201. A single straight groove for the blade portion 202 and a pair of recesses which are of a semi-spherical surface are cut in the jawbone by a cutting tool as shown in FIG.19 in which relatively thin, small diameter unit cutter disks 17 of varying diameters are securely clamped together and mounted on the rotary shaft 18 on both sides of a relatively thick, large diameter cutter disk 10 to collectively form a semi-spherical cutting surface.

The principles of the use of thin unit cutter disks to provide a predetermined shaped cutting surface can be generalized to provide a cutting tool and apparatus which in principles have wide applicabilities. For example, variously shaped cutter edge surfaces may be required in not only dental but medical surgical operations. Further, in wood cutting, profiled cutters are desired to provide ornamental profiled wood surfaces.

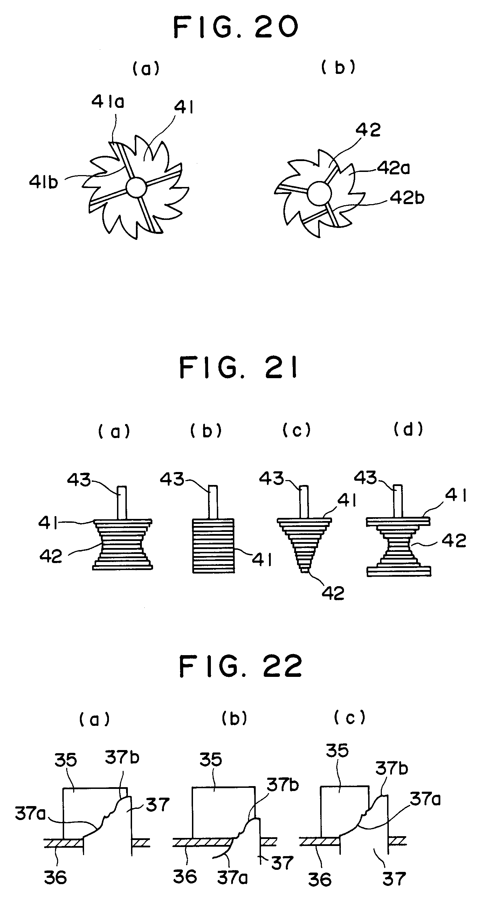

In conventional ornamental wood cutting processes, a single profiled cutter may be used to cut various profiles in wood materials by changing particular profile portions of the single cutter. This is illustrated with reference to FIG.22 in which a wood blank 35 is shown as mounted on and slidably displaceable over a worktable 36 for ornamental machining by a cutter 37 with an overall cutting edge profile consisting of different profile portions 37a and 37b . The particular cutter 37 shown can be secured to the worktable 36 to take one of three working positions. Thus, in a cutting operation shown in FIG.22(a), the cutter 37 is secured to the table 36 to take its cutting position with its overall cutting edge profile 37a and 37b projecting above the table 36 and effective to machine the wood blank 35 with these projected cutter edge profiles. In the stage shown in FIG.22(b), only the cutter profile portion 37b is effective to act on the blank 35. In the further stage shown in FIG.22(c), the other cutter profile portion 37a is effective with respect to the blank 35 to be machined. The cutter 37 is not effective to cut profiles other than profiles complementary to the particular cutter profiles 37a or 37b.

FIGS.21(a)-(d) show cutting tools prepared by stacking a plurality of unitary cutter disks of an identical or varying thickness and diameters with numeral 41 representing disks of larger diameters and numeral 42 representing disks of smaller diameters. Each individual disk 41, 42, as mentioned previously, preferably has a peripheral cutter edge zone (annular) provided with an added thin larger of hard material which may be tungsten carbide (WC), titanium carbide (TiC), boron carbide (B$_4$C), silicon carbide (SiC), alumina (Al$_2$O$_3$), zirconia (ZrO) or titanium nitride (TiN), preferably with or without diamond or CBN particles added thereto. For the formation of such an added layer, the spark deposition, PVD or CVD techniques may be advantageously employed as described previously. Thus, referring to FIG.20, the wear-resistant properties of the disk edge portions 41a, 42a are markedly improved, also permitting the base disk portion to be composed of a soft and suitable material such as stainless steel. Furthermore, overall cutting performance is significantly enhanced with each unitary cutter disk 41, 42 having such an added hard material coating.

FIGS.20(a) and 20(b) further show provision of fluid passages 41b, 42b formed in or on the disk 41, 42 for guiding and flashing a coolant into the cutting zone. A particular coolant is selected depending upon the nature of a cutting operation performed. In bone cutting in a medical or dental surgery, a salt solution or a sterilizing gas coolant is recommended as previously described.

In FIGS.21(a)-(d), unit cutter disks 41, 42 are variously combined for tightly clamping together Upon the rotary shaft 43 to provide a variety of shaped cutter profiles. The example of FIG.21(a) has large diameter unit disks 41 positioned at both two extremities and smaller diameter unit disks 42 stacked together therebetween to provide a parabolic cutting surface. The example of FIG.21(b) makes use of unit cutter disks 41 or 42 of an identical diameter to form a cylindrical cutting surface. FIG.21(c) shows an example of the cutting tool in which unit disks 41, 42 are combined to form a conical cutting surface. The cutting tool of FIG.21(d) utilizes two large diameter unit disks 41 of an identical diameter at each opposite end and smaller unitary disks 42 of varying diameters are assembled between the large unit disks 41 to provide a sharply convexed or parabolic machining surface.

In practice, one may simply prepare a reasonable number of unit or elemental disk, 41, 42, of identical and various diameters and, depending on a particular profile to be cut in a work blank, may choose a set of such disks and assemble and clamp them upon the rotary shaft 43 to provide a cutting surface complementary to the profile to be cut in the blank or bone. It will be seen that a vast number of combinations is possible which is far greater than the number of unit or elemental disks 41, 42 prepared.

Sometimes it is desirable to provide an interstice or small spacing between adjacent disks 41, 42 particularly at their cutting edge portions 41a, 42a, to facilitate removal of cutting chips and flashing of the cutting areas with a coolant. Such interstices may be provided by interposing a small ring between the adjacent disks.

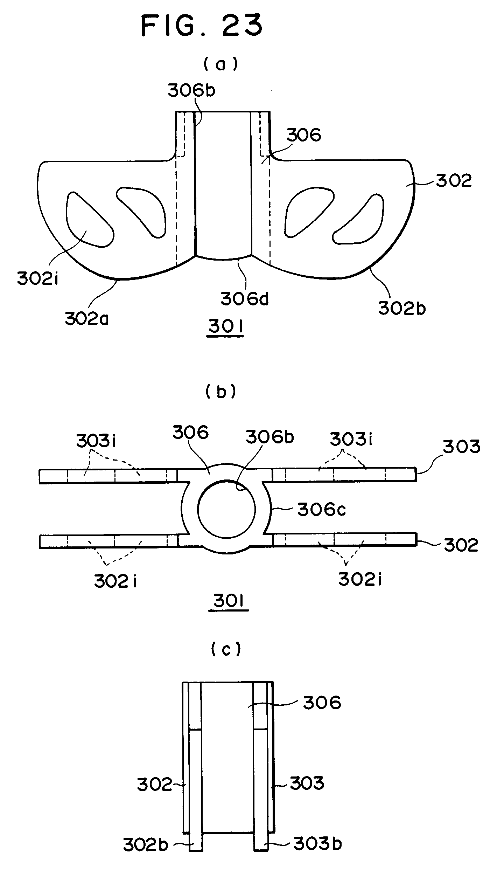

FIGS.23(a)-(c) show another form of the root and head base portion of the dental implant 301 to which the principles of the invention may apply. The blade-type implant 301 here again includes a pair of blade portions 302, 303 which are parallel connected and supported by the base head portion 306 which is singular here supporting an implant head (not shown). The latter is here adapted to be threaded internally into the base portion 306. The base portion 366 thus is formed with threads along its inner cylindrical bore 306b and thus an internally threaded hollow cylinder whose outer surface is shown at 306c. Each blade portion again is provided with vents 302i and has a double-arc profiled rim constituted by a pair of co-planar arc-formed contours 302a and 302b. These contours are separated by a curved bottom surface 306d of the cylindrical base member 306, which surface is concentric with an arc shown by a broken line 51. The arc 15 drawn represents a single, tangential arc extension of the curved blade profile from its opposite sides co-planarly containing two arcs 302a 306c insides.

The cutting tool and apparatus 15, previously shown and described, is here utilized to form in the bone a pair of parallal grooves, each for accepting the arc 51 and two arcs 302a and 302b; 303a and 303b slightly thereabove, and further a recess for accepting the surface 306b of the head base portion 306.

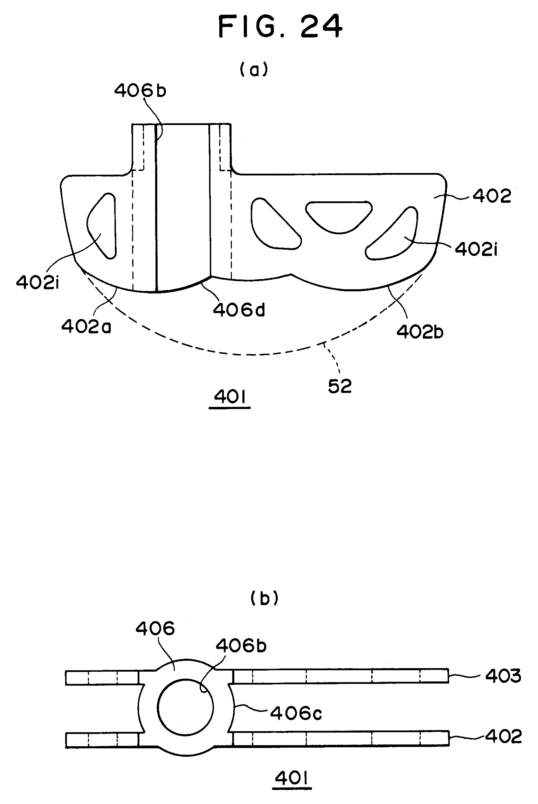

FIGS.24(a)-(b) show a further modified form of the implant 401 with a pair of double-arc profiled blade portions 402 and 403 similar to the form shown in FIGS.23(a)-(c). In the examples of FIGS.24(a)-(d), however, the position of the head base portion 406 is deviated towards one side of the assembly and blade portions 402a and 402b; and 403a and 403b are asymmetrical with respect to the head base portion 406. In other words, one arc blade portion 402a; 403a partially overlaps and is continuous with the curved end surface 406d of the hollow cylindrical head base portion 406. In any case, an imaginary envelop arc 52 is provided, and the cutting tool and apparatus previously shown and described may be used to form a pair of parallel grooves, each for accepting the arc 52 and surfaces 402a, 406d and 402b; 403a, 406d and 403b.

## Claims

**1.** A dental bone cutting tool for machining recesses for anchoring a dental implant in a jawbone in which the implant comprises a plurality of blade members juxtaposed and connected in a parallel relationship with one another and a head member adapted to support a denture or denture supporting structure, and supported by the blade members, and having a base surface continuous therewith, characterized in that the tool comprises:

a plurality of relatively large diameter cutter disks constituting each a circular saw blade with a peripheral cutter edge and mounted securely on a rotary shaft for cutting a plurality of recesses for accepting said implant blade members in the jawbone; and

a predetermined number of relatively small diameter cutter disks clamped in parallel together and mounted on said rotary shaft and which are so numbered and individually sized as to collectively constitute a substantially unitary saw blade to provide a profiled cutting surface with their peripheral cutting edges for cutting a recess so sized and configured as to accept at least a portion of the base surface of said implant head member in said jawbone.

2. The bone cutting tool defined in claim 1 characterized in that said relatively small diameter cutter disks are thin, and unit cutter disks of an equal thickness are prepared in a set with various diameters.

3. The bone cutting tool defined in claim 1 or claim 2 characterized in that said rotary shaft is hollow to provide a fluid passage for flushing a coolant in a cutting zone constituted by said peripheral cutting edges.

4. The bone cutting tool defined in claim 1 or claim 2 characterized in that said implant blade members are composed of a bio-compatible substance and said large diameter disks have individually a thin annular layer of a hard material containing said bio-compatible substance coated thereon along a region of a said peripheral cutting edge.

5. The bone cutting tool defined in claim 4 characterized in that said bio-compatible substance consists substantially of pure titanium or a titanium alloy and said material consists substantially of titanium nitride or titanium carbide and said cutter disks have individually a base body or substrate consisting of a soft stainless steel.

6. A dental cutting tool for machining recesses for anchoring a dental implant in a jawbone in which the implant comprises a blade member and at least one head member adapted to support a denture or denture supporting structure and supported by the blade member, and having a base surface continuous therewith, characterized in that the tool comprises:

a relatively large diameter cutter disk constituting a circular saw blade with a peripheral cutting edge and mounted securely on a rotary shaft for cutting a recess for accepting said implant blade member in the jawbone; and

a predetermine number of relatively small diameter cutter disks clamped together and with said relatively large diameter cutter disk and mounted on said rotary shaft as being divided on both side of said relatively large diameter cutter disk, the relatively small diameter cutter disks being so numbered and individually dimensioned and configured to accept at least a portion of the base surface of said implant head member in said jawbone.

7. The bone cutting tool defined in claim 6 characterized with said relative small diameter cutter disks are thin and said unit cutter disks of an equal thickness are prepared in a set with various diameters.

8. The bone cutting tool defined in claim 6 or claim 7 characterized with said rotary shaft is hollow and formed with an internal fluid passage communicating with a cutting zone constituted by said peripheral edges for flushing said zone with said coolant.

9. The bone cutting tool defined in claim 6 or claim 7 characterized with said implant blade member is composed of a bio-compatible substance and said large diameter disk has an annular layer of a hard material containing said bio-compatible substance coated thereon along a region of said peripheral cutting edge.

10. The bone cutting tool defined in claim 9 characterized with said small diameter disks have individually an annular layer of a hard material containing said bio-compatible substance coated thereon along a region of a said peripheral cutting edge.

11. The bone cutting tool defined in claim 9 or 10 wherein said bio-compatible substance is pure titanium or a titanium alloy and said hard material is titanium nitride or titanium carbide, and said cuter disks have individually a base body or substrate consisting of a soft stainless steel.

12. A dental bone cutting apparatus for machining recesses in a jawbone for anchoring a dental implant comprising at least one blade member, and at least one head member form supporting a denture or a denture supporting structure, said at least one head member having a predetermined base surface profile and being supported by said at least one blade member and continuous therewith, characterized in that the apparatus comprises:

at least one relatively large diameter cutter disk constituting a first circular saw blade means with peripheral cutter edges thereof for cutting said at least one implant blade member;

a plurality of relatively small diameter cutter disks clamped together and with said at least one relatively large diameter cutter disk on a rotary shaft, and so numbered and dimensioned as to constitute a substantially unitary rotary saw blade with their peripheral cutting edges for cutting thereby a recess so dimensioned as to accept said predetermined profile base surface of said at least one head member;

said rotary shaft being hollow and having an internal fluid passage;

drive means for rotating said hollow rotary shaft; and

a coolant supply for supplying a coolant under pressure into said fluid passage of the rotary shaft, when rotated at a high speed, for flushing the coolant into a bone cutting zone constituted by said cutter saw blades while in rapid rotation.

13. A bone cutting apparatus for at least one recess of a predetermined profile in a patient bone in a surgical operation, characterized in that it comprises:

a plurality of thin, unit cutter blades clamped in parallel together and mounted on a hollow rotary shaft, the unit cutter blades being so numbered and dimensioned as to collectively constitute a substantially unitary rotary saw blade with a cutting profile substantially complementary to said predetermined profile;

said hollow rotary internally having a fluid passage communicating with a cutting zone constituted by said cutter blades;

drive means for rotating said hollow rotary shaft and thereby said substantially unitary rotary blade for cutting said recess; and

means for supplying a fluid coolant through said fluid passage into said cutting zone.

14. A method of cutting a contour of a predetermined profile in a workpiece, characterized by comprising the steps of:

preparing a plurality of thin, unit cutter disks of various diameters;

selecting a predetermined set of said unit cutter disks, arranging and clamping together and upon a rotary shaft in such a manner that said selected, arranged and clamped disks collectively constitute a substantially unitary rotary cutter with a profile substantially complementary to said predetermined profile; and

rotating said rotary shaft and thereby said clamped disks for cutting said contour in said workpiece.

15. The method defined in claim 14 characterized in that said rotary shaft is hollow and provided with an internal fluid passage communicating with a cutting zone constituted by said disks and said workpiece, further comprising the step of:

passing a coolant through said fluid passage to flush said cutting zone therewith.

16. A bone cutting tool characterized by comprising a rotary cutter disk composed of a soft material and having an added annular layer of a hard material containing a bio-compatible substance coated along a peripheral cutting region of said disk.

17. The bone cutting tool defined in claim 16 characterized in that said substance is titanium.

18. The bone cutting tool defined in claim 17 characterized in that said hard material consists of titanium nitride or titanium carbide.

19. The bone cutting tool defined in claim 16 characterized in that said material consists of a continuous body of titanium nitride or titanium carbide and fine particles of diamond or CBN distributed therein.

20. The tool defined in claim 16, claim 17 or claim 18, characterized in that said disk is formed with a plurality of radially extending fluid passages or guiding a coolant into a bone cutting zone.

21. A dental implant characterized by comprising:

a blade-type root portion comprising a pair of blade members juxtaposed in parallel with each other and connected by a head portion, the blade members having a vertical height $a$; and

said head portion having a lower end with a base contour of a predetermined profile, said head lower and extending into between said blade members with a length of extension $b$,
wherein $b$ is not greater than one half of $a$.

# FIG. 1

# FIG. 2

# FIG. 3

# FIG. 4

# FIG. 5

# FIG. 6

# FIG. 7

# FIG. 8

# FIG. 9

# FIG. 10

# FIG. 11

(a)

104d    101    105d    105c
104c
104
106    107
102    106a    107a
      105

D

102i    102j    102b
102a    B    102i
A
103a    106a    103j    107a    103b
                            103
                            102

102a    102j    102b
(c)

(b)

105c
105
107a
107
102    103
102b    103b

# FIG. 12

(b)    16    16a
9
18    17
    17a

(a)
17    17
16    15
16
18

# FIG. 13

16    17

17

17    15

16

16    17

18

# FIG. 14

17

B    16

19

20S    20

D

A

# FIG. 15

109

109a    110    111    109b

108a    108b

108

# FIG. 16

## FIG. 16 (a)

## FIG. 16(b)

## FIG. 16(c)

EP 0 517 529 A2

# FIG. 17

16    17    15

17

16    17

# FIG. 18

(a)    201    (b)    201

104c    104    105    105c
106    106a
202    107    107a    105    107a
D    202b    202
202i    202a    202i    202a
B
A

106a    107a
202
202a    202j    202b
(c)

# FIG. 19

# FIG. 20

(a)

(b)

# FIG. 21

(a)  (b)  (c)  (d)

# FIG. 22

(a)  (b)  (c)

# FIG. 23

( a )

306b

306

302

302i

306d

302a

302b

301

( b )

303i    306    306b    303i    303

306c

302

302i    302i

301

( c )

306

302    303

302b    303b

# FIG. 24

## (a)

## (b)